Europäisches Patentamt

European Patent Office

Office européen des brevets

(11) Veröffentlichungsnummer: **0 004 079**
**B1**

# EUROPÄISCHE PATENTSCHRIFT

(45) Veröffentlichungstag der Patentschrift: **17.06.81**

(21) Anmeldenummer: **79100702.4**

(22) Anmeldetag: **08.03.79**

(51) Int. Cl.³: **B 01 J 35/02,**
**C 07 C 67/05, C 07 C 69/15**

(54) Träger-Katalysator für die Herstellung von Vinylacetat aus Ethylen, Essigsäure und Sauerstoff in der Gasphase.

(30) Priorität: **15.03.78 DE 2811115**

(43) Veröffentlichungstag der Anmeldung:
**19.09.79 Patentblatt 79/19**

(45) Bekanntmachung des Hinweises auf die Patenterteilung:
**17.06.81 Patentblatt 81/24**

(84) Benannte Vertragsstaaten:
**BE DE FR GB IT NL**

(56) Entgegenhaltungen:
**DE - A - 2 544 185**
**DE - B - 1 277 249**
**US - A - 2 644 800**
**US - A - 3 764 565**

(73) Patentinhaber: **HOECHST AKTIENGESELLSCHAFT**
**Zentrale Patentabteilung Postfach 80 03 20**
**D-6230 Frankfurt/Main 80 (DE)**

(72) Erfinder: **Wunder, Friedrich, Dr.**
**Jahnstrasse 46**
**D-6093 Flörsheim am Main (DE)**
Erfinder: **Quadflieg, Therese, Dr.**
**Mainblick 14**
**D-6233 Kelkheim (Taunus) (DE)**
Erfinder: **Roscher, Günter, Dr.**
**Altkönigstrasse 7**
**D-6233 Kelkheim (Taunus) (DE)**
Erfinder: **Heck, Günther, Dipl.-Ing.**
**Buchenweg 1**
**D-6200 Wiesbaden (DE)**

Courier Press, Leamington Spa, England.

**0 004 079**

Träger-Katalysator für die Herstellung von Vinylacetat aus Ethylen, Essigsäure und Sauerstoff in der Gasphase.

Es ist bekannt, daß man Ethylen mit Essigsäure und Sauerstoff in der Gasphase zu Vinylacetat umsetzen kann. Geeignete Katalysatoren enthalten einen Edelmetallanteil und einen Aktivatoranteil. Der Edelmetallanteil besteht aus Edelmetallen der 8. Nebengruppe des Periodensystems und/oder deren Verbindungen; zusätzlich können noch Elemente der 1. Nebengruppe und/oder deren Verbindungen anwesend sein. Der Aktivatoranteil besteht aus Verbindungen von Elementen der 1. Hauptgruppe und/oder der 2. Hauptgruppe und/oder Cadmium. Bevorzugt ist Palladium als Element der 8. Nebengruppe, Gold als Element der 1. Nebengruppe, Kalium als Element der 1. Hauptgruppe. Diese aktiven Komponenten werden auf Träger in feiner Verteilung aufgebracht, wobei als Trägermaterial im allgemeinen Kieselsäure oder Aluminiumoxid verwendet wird. Jedoch müssen zum Erreichen von sehr hohen Raum-Zeit Leistungen die Träger-Katalysatoren einen relative hohen Gehalt an Palladium und gegebenenfalls zusätzlich Gold haben, was einen erheblichen wirtschaftlichen Aufwand bedeutet. Mit Katalysatoren, bei denen nur die äußere Schicht des Trägers mit den aktiven Komponenten imprägniert ist, lassen sich im allgemeinen keine höheren Raum-Zeit-Leistungen als 300—500 g Vinylacetat je Liter Katalysator und Stunde erreichen. Mit Katalysatoren, bei denen das gesamte Trägermaterial imprägniert ist, werden Raum-Zeit-Leistungen von 1000—1200 g/l·h erreicht, wobei jedoch ein höherer Edelmetall-Einsatz notwendig ist. Bei dem in vielen Anlagen großtechnisch ausgeübten Verfahren würde ein Katalysator, der mit geringerem Edelmetall-Einsatz gleiche oder größere Raum-Zeit-Leistungen erbringt, einen großen wirtschaftlichen Vorteil bedeuten.

Es wurde nun ein Katalysator für die Herstellung von Vinylacetat in der Gasphase aus Ethylen, Essigsäure und Sauerstoff oder Sauerstoff enthaltenden Gasen gefunden, der Edelmetalleder 8. Nebengruppe und/oder deren Verbindungen, sowie gegebenenfalls zusätzlich Gold und/oder Goldverbindungen, sowie als Aktivatoren Alkaliverbindungen und/oder Erdalkaliverbindungen und/oder Cadmiumverbindungen auf einem Träger enthält, dadurch gekennzeichnet, daß der Träger aus Strangabschnitten mit sternförmigem Querschnitt oder gerippten Strängen besteht. Vorzugsweise verwendet man Strangabschnitte mit sternförmigem Querschnitt.

Im allgemeinen werden in der Katalyse Träger eingesetzt, die aus Teilchen mit großen äußeren Oberflächen bestehen, wie z.B. Kugeln mit rauher Oberfläche, Kugeln mit aufgesinterten Partikeln, Stränge, Zylinder, Tabletten und Raschigringe. Hierbei wird häufig eine Leistungssteigerung der Katalysatoren gegenüber der reinen Kugelform beobachtet, die bei Raschigringen bis zu 20% erreichen kann. Es war daher zu erwarten, daß auch bei der Umsetzung von Ethylen, Essigsäure und Sauerstoff zu Vinylacetat durch Verwendung von Trägerteilchen mit größerer äußerer Oberfläche, wie z.B. Raschigringen, eine Leistungssteigerung eintritt. Bei entsprechenden Versuchen (Vergleichsbeispiele 1 bis 4) zeigte sich jedoch, daß dies nicht der Fall ist. Bei Verwendung von Raschigringen trat gegenüber kugelförmigen Trägerteilchen eine Leistungsminderung ein; bei Zylinder-Strängen und Tabletten konnte die Leistung der Kugeln erreicht werden, wobei sich jedoch ein Druckabfall zeigte, der sich sehr störend auswirkte. Es ist nun außerordentlich überraschend, daß Träger, die aus Strangabschnitten mit sternförmigem Querschnitt — im folgenden "Sternstränge" gennant — bestehen, relativ zum Schüttgewicht (= Masse von 1 Liter geschüttetem Trägermaterial) und damit zur eingesetzten Palladiummenge eine etwa doppelt so hohe Leistung erbringen wie ein kugelförmiges Trägermaterial. Das Schüttgewicht der Sternstränge ist nämlich nur etwa halb so groß wie das Schüttgewicht bei kugelförmigen Trägerteilchen. Daraus folgt, daß (bei gleicher Palladiumkonzentration im einzelnen Trägerteilchen) in 1 Liter geschüttetem Sternstrang—Träger nur etwa die Hälfte der Palladiummenge vorhanden ist, wie in 1 Liter geschüttetem Kugel-Träger. Trotzdem ist die Raum-Zeit-Leistung (= Menge an Vinylacetat pro Liter geschüttetem Träger und pro Stunde) gleich. Es wird also die gleiche Raum-Zeit-Leistung mit der halben Palladiummenge erreicht. Dies ist besonders deswegen überraschend, weil die äußere Oberfläche bei Sternsträngen kleiner ist als bei Kugeln (beides bezogen auf 1 l geschütteten Katalysator).

Außerdem ist der Druckabfall bei Sternstrang-Trägern sogar noch geringer als bei Kugel-Trägern, was eine Energieeinsparung bedeutet. Das Analoge gilt für gerippte Stränge ("Rippenstränge"). Die Stern- oder Rippenstränge können aus allen in der Literatur für diese Umsetzung als Trägermaterial bekannten Substanzen bestehen. Beispielsweise seien Kieselsäure, Kieselgel, Silikate (etwa solche des Aluminiums, Titans, Zirkons, Berylliums, Magnesiums, der seltenen Erden), gemischte Silikate (wie Tonmineralien, Feldspäte) sowie Aluminiumoxide, Spinelle, Titanoxid, Zirkonoxid, Kohlenstoff in seinen verschiedenen Formen (wie Aktivkohle, Koks und Graphit) und Siliciumcarbid genannt. Bevorzugt sind jedoch Kieselsäure, Kieselgel, Spinelle, aluminiumsilikate, Tonmineralien, Aluminiumoxid. Besonders bevorzugt ist Kieselsäure. Die Abmessung der Stern- oder Rippenstränge wird vorzugsweise so gewählt, daß einerseits ein leichtes Einfüllen des Trägers in den Reaktor gewährleistet ist (d.h. Ausschluß extrem großer Teilchen) und andererseits kein großer Druckabfall entsteht (d.h. Ausschluß extrem kleiner Teilchen). Im allgemeinen haben die Stränge Durchmesser von 3—15 mm, bevorzugt von 4—7 mm (bezogen auf den gedachten, die Stränge umschließenden Zylinder). Die Sterne haben mindestens 3 und im allgemeinen bis zu 15 Zacken, wobei 4 bis 6-zackige (oder -zählige) Sterne bevorzugt sind. Die Länge der Strangabschnitte ist vorzugsweise geringer als der innere Durchmesser des

0 004 079

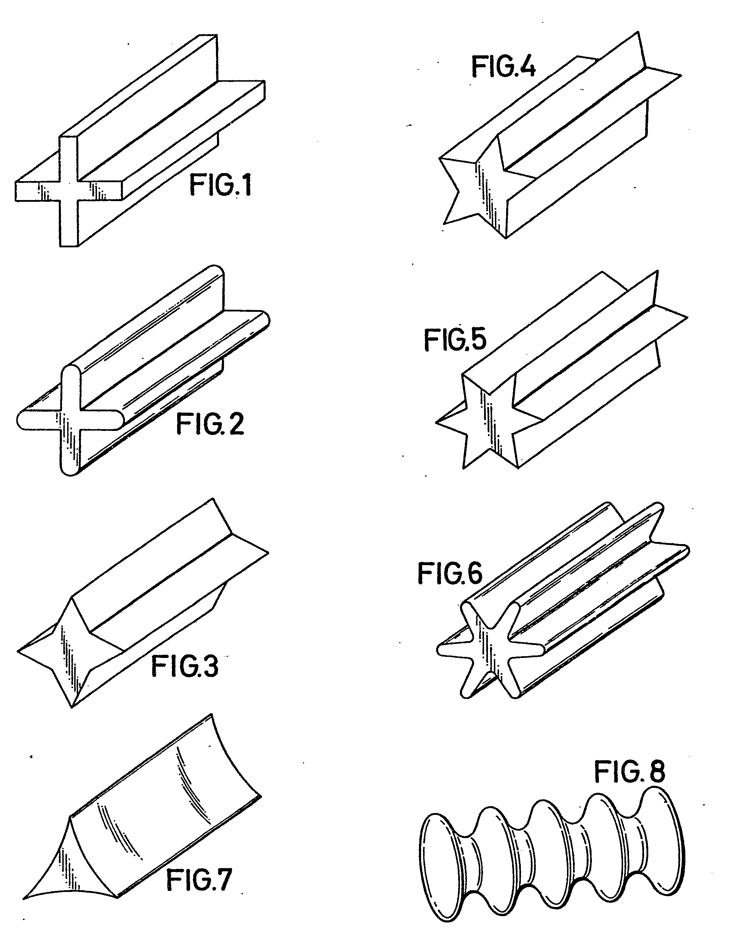

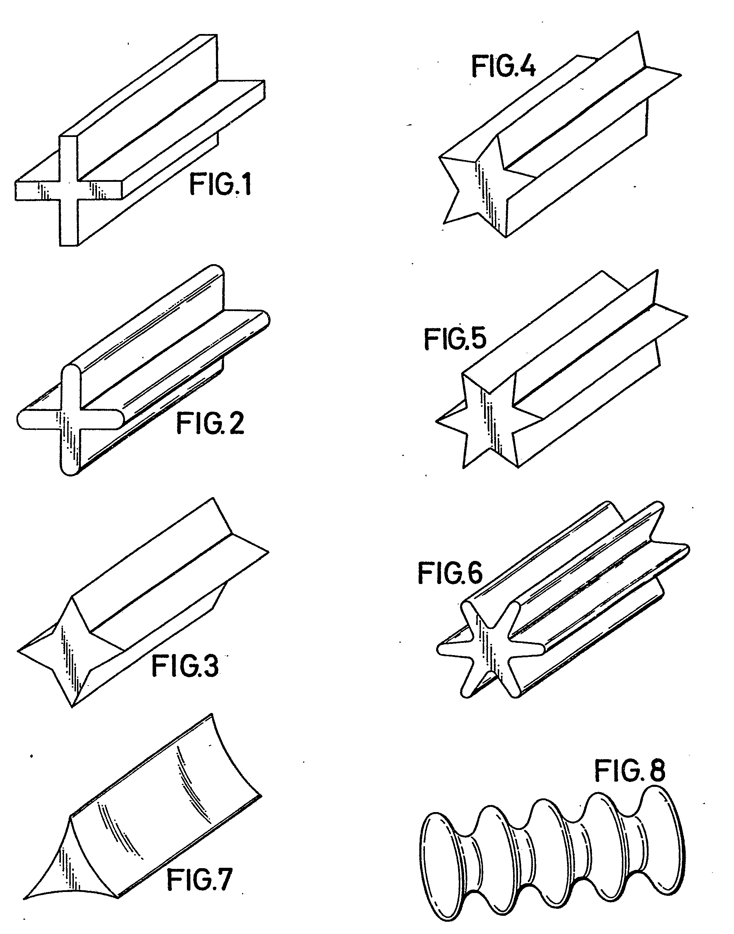

Reaktorrohrs (bzw. der Reaktorrohre), meist liegt sie zwischen 4 und 20 mm, kann jedoch auch größer oder kleiner sein; bevorzugt werden Stränge zwischen 6 und 15 mm Länge. Die Tiefe der Einschnitte und damit der Durchmesser des Strangkernes sollte so gewählt werden, daß eine ausreichende mechanische Festigkeit gewährleistet ist. Im allgemeinen hat dieser Kern einen Durchmesser von 2 bis 4 mm, kann jedoch in besonderen Fällen wesentlich kleiner oder größer sein. Die Form der Sternzacken bzw. Rippen kann eckig oder gerundet sein. Die Figuren 1 bis 8 zeigen einige geeignete Formen:

Figur 1: 4-zähliger Sternstrang mit Rechteck-Zacken
Figur 2: 4-zähliger Sternstrang mit gerundeten Zacken
Figur 3: 4-zähliger Sternstrang mit Dreieck Zacken
Figur 4: 5-zähliger Sternstrang mit Dreieck-Zacken
Figur 5: 6-zähliger Sternstrang mit Dreieck-Zacken
Figur 6: 6-zähliger Sternstrang mit Zahnrad-Zacken (ellipsoidisch)
Figur 7: 3-zähliger Sternstrang mit eckigen Zacken
Figur 8: gerippter Strang mit gerundeten Rippen
Bevorzugt sind jedoch Dreieck-Zacken (Figur 3, 4, 5).

Die Herstellung der Stern- oder Rippenstränge erfolgt in bekannter Weise durch Extrusion, Pressen in einer Form (Tablettenpresse mit entsprechender Matrix) oder Ausgießen von Hohlformen. Bevorzugt ist jedoch die Extrusion.

Die katalytisch aktiven Substanzen werden in üblicher Weise auf den Träger aufgebracht, beispielsweise durch Tränken des Trägers mit einer Lösung der aktiven Substanzen, anschließende Trocknung und gegebenenfalls Reduktion. Jedoch kann man die aktiven Substanzen auch beispielsweise durch Ausfällung auf dem Träger, durch Aufsprühen, Aufdampfen, Tauchen aufbringen.

Als Lösungsmittel für die katalytisch aktiven Substanzen sind vor allem unsubstituierte Carbonsäuren mit bis zu 10 Kohlenstoffatomen im Molekül, wie Essigsäure, Propionsäure, n- und iso-Buttersäure und die verschiedenen Valeriansäuren geeignet. Wegen ihrer physikalischen Eigenschaften und auch aus wirtschaftlichen Gründen wird vorzugsweise Essigsäure als Lösungsmittel eingesetzt. Die zusätzliche Verwendung eines inerten Lösungsmittels ist dann zweckmäßig, wenn die Substanzen in der Carbonsäure nicht ausreichend löslich sind. so läßt sich z.B. Palladiumchlorid in einer wäßrigen Essigsäure wesentlich besser lösen als in Eisessig. Als zusätzliche Lösungsmittel kommen diejenigen in Betracht, die inert und mit der Carbonsäure mischbar sind. Genannt seien neben Wasser beispielsweise Ketone wie Aceton und Acetylaceton, ferner Äther wie Tetrahydrofuran oder Dioxan, aber auch Kohlenwasserstoffe wie Benzol.

Als Verbindungen von Edelmetallen der 8. Nebengruppe kommen alle Salze und Komplexe in Betracht, die löslich (sowie gegebenenfalls reduzierbar) sind und im fertigen Katalysator keine desaktivierenden Stoffe wie Halogen oder Schwefel hinterlassen. Besonders geeignet sind die Carboxylate, vorzugsweise die Salze der aliphatischen Monocarbonsäuren mit 2 bis 5 Kohlenstoffatomen, etwa das Acetat, das Propionat oder das Butyrat. Weiter sind beispielsweise geeignet die Nitrate, Nitrite, Oxidhydrate, Oxalate, Acetylacetonate, Acetoacetate. Aber auch Verbindungen wie die Sulfate und die Halogenide können verwendet werden, wenn man dafür Sorge trägt, daß der Sulfatrest, z.B. durch Fällen mit Bariumacetat, oder das Halogen, z.B. durch Fällen mit Silbernitrat, vor der Tränkung entfernt wird, so daß das Sulfat- oder Halogenanion nicht auf den Träger gerät. Bevorzugt verwendet man Palladium in Form seiner eben genannten Verbindungen. Wegen seiner Löslichkeit und seiner Zugänglichkeit ist Palladiumacetat die besonders bevorzugte Palladiumverbindung.

Im allgemeinen liegt der Gehalt an Elementen der 8. Nebengruppe im Katalysator zwischen 0,5 und 5 Gew.-%, wobei der Metallanteil auf die Gesamtmasse des Trägerkatalysators bezogen wird.

Neben Edelmetallen der 8. Nebengruppe und/oder ihren Verbindungen kann noch zusätzlich Gold und/oder eine seiner Verbindungen anwesend sein. Eine besonders geeignete Goldverbindung ist Bariumacetoaurat.

Im allgemeinen wird Gold bzw. eine seiner Verbindungen, falls es eingesetzt wird, in einem Anteil von 0.01 bis 4 Gew. % zugegeben, wobei der Metallanteil auf die Gesamtmasse des Trägerkatalysators bezogen wird.

Als Aktivatoren enthält der Katalysator Alkaliverbindungen und/oder Erdalkaliverbindungen und/oder Cadmiumverbindungen. Geeignet sind beispielsweise Alkalicarboxylate und Erdalkalicarboxylate, wie etwa Kaliumacetat, Natriumacetat, Lithiumacetat, Natriumpropionat, Calciumisobutyrat, Magnesiumacetat; geeignet sind auch solche Alkali- oder Erdalkaliverbindungen, die unter Reaktionsbedingungen in die Carboxylate übergehen, wie etwa Hydroxide, Oxide, Carbonate. Als Verbindungen des Cadmiums kommen solche in Frage, die kein Halogen oder Schwefel enthalten, beispielsweise Carboxylat(bevorzugt), Oxid, Hydroxid, Carbonat, Citrat, Tartrat, Nitrat, Acetylacetonat, Benzoylacetonat, Acetoacetat. Besonders geeignet ist Cadmiumacetat. Auch Gemische verschiedener Aktivatoren lassen sich einsetzen. Jeder einzelne Aktivator wird im allgemeinen in einem Anteil von 0,01 bis 4 Gew.-% zugegeben, wobei der Metallanteil des Aktivators auf die Gesamtmasse des Trägerkatalysators bezogen wird.

Bevorzugt sind folgende Katalysatoren:

Palladium/Alkali/Cadmium sowie Palladium/Gold/Alkali, wobei Palladium bzw. Gold als Metalle

# 0 004 079

oder Verbindungen im fertigen Katalysator vorliegen können und wobei als Alkalielement Kalium bevorzugt ist (in Form eines Carboxylats).

Besonders bevorzugt sind die Katalysatoren Palladiumacetat/Kaliumacetat/Cadmiumacetat, sowie Palladiumacetat/Bariumacetoaurat/Kaliumacetat.

Die Tränkung des Katalysatorträgers mit der Lösung der aktiven Komponenten wird vorzugsweise so vorgenommen, daß das Trägermaterial mit der Lösung überschichtet und die überschüssige Lösung dann abgegossen oder abfiltriert wird. Mit Rücksicht auf Lösungsverluste ist es vorteilhaft nur die dem integralen Porenvolumen des Katalysatorträgers entsprechende Lösung einzusetzen und sorgfältig durchzumischen, damit alle Teilchen des Trägermaterials gleichmäßig benetzt werden. Diese Durchmischung läßt sich z.B. durch Rühren erreichen. Es ist zweckmäßig, den Tränkungsvorgang und das Durchmischen gleichzeitig durchzuführen, beispielsweise in einer Drehtrommel oder einem Taumeltrockner, wobei sich die Trocknung sofort anschließen kann. Weiterhin ist es zweckmäßig, die Menge und die Zusammensetzung der zum Tränken des Katalysatorträgers verwendeten Lösung so zu bemessen, daß sie dem Porenvolumen des Trägermaterials entspricht und daß durch einmaliges Tränken die gewünschte Menge aktiver Stoffe aufgebracht wird.

Die Trocknung des mit der Lösung der aktiven Stoffe getränkten Katalysatorträgers wird vorzugsweise unter vermindertem Druck durchgeführt. Weiterhin empfiehlt es sich im allgemeinen, die Trocknung in einem Inertgasstrom, beispielsweise in einem Stickstoff- oder Kohlendioxidstrom vorzunehmen. Der Lösungsmittel-Restgehalt beträgt vorzugsweise weniger als 8 Gew.-%, vorzugsweise weniger als 6 Gew.-%.

Falls eine Reduktion der Edelmetallverbindungen (und ggf. der Goldverbindungen) durchgeführt wird, so kann diese im Vakuum, bei Normaldruck oder bei erhöhtem Druck bis zu 10 bar ausgeführt werden. Dabei empfiehlt es sich, das Reduktionsmittel umso stärker mit einem Inertgas zu verdünnen, je höher der Druck ist. Die Reduktionstemperatur liegt zwischen 40 und 260°C, vorzugsweise zwischen 70 und 200°C. Im allgemeinen ist es zweckmäßig, für die Reduktion ein Inertgas-Reduktionsmittel-Gemisch zu verwenden, das 0,01 bis 50 Vol.-%, vorzugsweise 0,5 bis 20 Vol.-% Reduktionsmittel enthält. Als Inertgas können beispielsweise Stickstoff, Kohlendioxid, Edelgase oder Paraffinkohlenwasserstoffe wie Methan, Äthan, Propan, Isobutan und Butan verwendet werden. Als Reduktionsmittel kommen beispielsweise Wasserstoff, Methanol, Formaldehyd, Äthylen, Propylen, Isobutylen, Butylen und andere Olefine in Frage. Die Menge des Reduktionsmittels richtet sich nach dem Oxydationsäquivalent des Edelmetalls der 8. Nebengruppe und gegebenenfalls des eingesetzten Goldes; das Reduktionsäquivalent soll mindestens das 1- bis 1,5-fache des Oxydationsäquivalents betragen, jedoch schaden größere Mengen Reduktionsmittel nicht. Beispielsweise soll auf 1 Mol Palladium mindestens 1 Mol Wasserstoff verwendet werden. Die Reduktion kann im Anschluß an die Trocknung in der gleichen Anlage vorgenommen werden.

Die Herstellung von Vinylacetat erfolgt im allgemeinen durch Leiten von Essigsäure, Ethylen und Sauerstoff oder Sauerstoff enthaltenden Gasen bei Temperaturen von 100 bis 250°C, vorzugsweise 120 bis 220°C, und bei Drucken von 1 bis 25 bar, vorzugsweise 1 bis 20 bar, über den fertigen Katalysator, wobei nicht-umgesetzte Komponenten im Kreise geführt werden können. Dabei ist es zweckmäßig, die Konzentrationsverhältnisse so zu wählen, daß das Reaktionsgemisch außerhalb der bekannten Explosionsgrenzen liegt. Zweckmäßig hält man die Sauerstoffkonzentration unter 8 Vol.-% (bezogen auf das essigsäurefreie Gasgemisch). Unter Umständen ist jedoch auch eine Verdünnung mit inerten Gasen, wie Stickstoff oder Kohlendioxid vorteilhaft. Besonders $CO_2$ eignet sich zur Verdünnung bei Kreisprozessen, da es in geringen Mengen während der Reaktions gebildet wird.

Die folgenden Beispiele sollen die Erfindung erläutern.

*Vergleichsbeispiel 1* (kugelförmige Trägerteilchen)

5,0 1 = 2550 g eines Kieselsäureträgers in Kugelform (6 mm Kugeldurchmesser) mit einer BET-Oberfläche von 120 m²/g, einem Schüttgewicht von 0,53 kg/l und einer äußeren Oberfläche von 0,81 m²/l (Definitionen s. Tabelle) wird

mit einer Lösung von

143 g Pd-acetat (47,3% Pd)

117 g Cd-acetat

133 g K-acetat

in 1780 ml Eisessig getränkt und bei 60°C unter Stickstoff bei einem Druck von 270 mbar getrocknet.

Es werden 4,5 l Katalysator in ein Reaktionsrohr von 30 mm lichter Weite und einer Länge von 7 m gefüllt.

Man leitet bei einem Druck von 9 bar (Reaktoreingang) und einer Katalysator-Temperatur von 175—178°C einen Gasstrom von 20,25 Nm³-h über den Katalysator. Der Gasstrom besteht vor dem Reaktor aus 60,8 Vol.% Ethylen, 15,5 Vol.% Inertgasen ($N_2$ und $CO_2$), 17.4 Vol.% Essigsäure und 6,3 Vol.% Sauerstoff.

Die Ergebnisse sind aus der Tabelle ersichtlich.

*Vergleichsbeispiel 2* (zylinderförmige Träger teilchen)

Es werden 2200 g $SiO_2$-Zylinderstränge mit einem Durchmesser von 6 mm und einer mittleren Länge von 8 mm, einer BET-Oberfläche von 190 m²/g, einem Schüttgewicht von 0,44 kg/l und einer äußeren Oberfläche von 1,47 m²/l

mit einer Lösung von

124 g Pd-acetat
100 g Cd-acetat
115 g K-acetat

in 1800 ml Eisessig getränkt und (wie in Vergleichsbeispiel 1) getrocknet. Ansonsten verfährt man wie in Vergleichsbeispiel 1. Die Ergebnisse sind aus der Tabelle ersichtlich.

*Vergleichsbeispiel 3* (Träger in Tablettenform)

Es werden 2600 g SiO₂-Tabletten mit zylindrischer Form (6 mm Durchmesser, 6 mm Höhe), einem Schüttgewicht von 0,5 kg/l, mit einer BET-Oberfläche von 148 m²/g und einer äußeren Oberfläche von 0,49 m²/l

mit einer Lösung von

144 g Pd-acetat
117 g Cd-acetat
133 g K-acetat

in 1780 Eisessig getränkt und (wie in Vergleichsbeispiel 1) getrocknet.

Ansonsten verfährt man wie in Vergleichsbeispiel 1. Die Ergebnisse sind aus der Tabelle ersichtlich.

*Vergleichsbespiel 4* (Träger in Form von Raschigringen)

Es werden 2600 g Raschigringe (4 mm Außendurchmesser, 1,5 mm Innendurchmesser, 6 mm Höhe) aus SiO₂ mit einer BET-Oberfläche von 200 m²/g, einem Schüttgewicht von 0,5kg/l, einer äußeren Oberfläche von 0,98 m²/l

mit einer Lösung von

144 g Pd-acetat
117 g Cd-acetat
133 g K-acetat

in 1025 ml Eisessig imgrägniert und getrocknet (wie in Vergleichsbeispiel 1). Ansonsten verfährt man wie in Vergleichsbeispiel 1. Die Ergebnisse sind aus der Tabelle ersichtlich.

Beispiel

Es werden 1377 g 5-zählige SiO₂-Sternstränge mit einer Zackentiefe von 1,7 mm, einem Durchmesser

von 6 mm (dies ist genau genommen der Durchmesser des gedachten engsten Zylinders, der die Sternstränge umschließt), einer mittleren Länge von 8 mm, Schüttgewicht 0,27 kg/l, äußerer Oberfläche 0,74m²/l, BET-Oberfläche 190 m²/g

mit einer Lösung von

78 g Pd-acetat
62 g Cd-acetat
73 g K-acetat

in 1583 ml Eisessig getränkt und getrocknet (wie in Vergleichsbeispiel 1). Ansonsten verfährt man wie in Vergleichsbeispiel 1. Die Ergebnisse sind aus der Tabelle ersichtlich.

Nach dem Trocknen enthalten alle 5 hergestellten Katalysatoren eine Dotierung von

2,3% Pd⁺⁺
1,7% Cd⁺⁺
1,9% K⁺

in Form der Acetate.

# 0 004 079

| | Vergleichs-beispiel 1 Kugeln | Vergleichs-beispiel 2 Zylinder-Stränge | Vergleichs-beispiel 3 Tabletten | Vergleichs-beispiel 4 Raschigringe | Beispiel Sternstränge |
|---|---|---|---|---|---|
| 1. BET-Oberfläche | 120 m²/g | 190 m²/g | 148 m²/g | 200 m²/g | 190 m²/g |
| 2. Äußere Oberfläche | 0,81 m²/l | 1,47 m²/l | 0,49 m²/l | 0,98 m²/l | 0,74 m²/l |
| 3. Schüttgewicht | 0,53 kg/l | 0,44 kg/l | 0,50 kg/l | 0,50 kg/l | 0,27 kg/l |
| 4. Palladiumgehalt | 13,5 g/l | 11,7 g/l | 13,6 g/l | 13,6 g/l | 7,3 g/l |
| 5. RZL | 1050 g/l.h | 1015 g/l.h | 1040 g/l.h | 872 g/l.h | 1067 g/l.h |
| 6. Spezifische Leistung | 77,8 g | 86,8 g | 76,5 g | 64,1 g | 146,2 g |
| 7. Staudruck | 0,31 bar/m | 0,36 bar/m | 0,49 bar/m | 0,26 bar/m | 0,23 bar/m |

1. Die "BET-Oberfläche" wird gemessen nach der Methode von Brunauer, Emmett und Teller und gibt die Gesamtoberfläche von 1 Gramm Trägermaterial an (d.h. die Summe aus äußerer Oberfläche und Oberfläche sämtlicher offener Poren).

2. Die "außere Oberfläche" des Trägers ist die Oberfläche von 1 Liter geschüttetem Trägermaterial, wobei die Oberfläche der Poren nicht mitgerechnet wird. Dieser Wert wird berechnet aus der Zahl der Trägerteilchen in 1 Liter geschüttetem Trägermaterial und der geometrischen Oberfläche eines Trägerteilchens.

3. Das "Schüttgewicht" ist die Masse von 1 Liter geschüttetem Trägermaterial.

4. Der "Palladiumgehalt" ist die Menge an Palladium in 1 Liter geschüttetem Trägermaterial.

5. "RZL" ist die Raum-Zeit-Leistung, d.h. die Menge an Endprodukt pro Liter geschüttetem Trägermaterial und pro Stunde.

6. Die "spezifische Leistung" ist die Menge an Vinylacetat pro Gramm Palladium und pro Stunde.

## Patentansprüche

1. Katalysator für die Herstellung von Vinylacetat in der Gasphase aus Ethylen, Essigsäure und Sauerstoff oder sauerstoffenthaltenden Gasen, der Edelmetalle der 8. Nebengruppe und/oder deren Verbindungen, sowie ggf. zusätzlich Gold und/oder Goldverbindungen, sowie als Aktivatoren Alkaliverbindungen und/oder Erdalkaliverbindungen und/oder Cadmiumverbindungen auf einem Träger enthält, dadurch gekennzeichnet, daß der Träger aus Stangabschnitten mit sternförmigem Querschnitt oder aus gerippten Strängen besteht.

2. Katalysator nach Anspruch 1, dadurch gekennzeichnet, daß der Querschnitt der Strangabschnitte aus einem 4 bis 6-zackigen Stern besteht.

3. Katalysator nach Ansprüchen 1—2, dadurch gekennzeichnet, daß der Träger aus Kieselsäure besteht.

## Revendications

1. Catalyseur pour la préparation de l'acétate de vinyle en phase gazeuse à partir de l'éthylène, de l'acide acétique et de l'oxygène, ou de gaz contenant de l'oxygène, qui contient, sur un support, des métaux nobles du 8 ème sous-groupe et/ou des composés de ces métaux nobles, en plus éventuellement de l'or et/ou des composés de l'or, et également, comme activateurs, des composés de métaux alcalins et/ou des composés de métaux alcalino-terreux et/ou des composés du cadmium, catalyseur caractérisé en ce que le support est constitué de tronçons de boudins à section transversale en forme d'étoile ou de boudins côtelés.

2. Catalyseur selon la revendication 1, caractérisé en ce que la section transversale des tronçons de boudins al la forme d'une étoile à 4, 5 ou 6 pointes.

3. Catalyseur selon l'une des revendications 1 et 2, caractérisé en ce que le support est constitué de silice.

## Claims

1. Catalyst for the preparation of vinyl acetate in the gaseous phase from ethylene, acetic acid and oxygen or oxygen-containing gases, which contains noble metals of the 8th sub-group and/or compounds thereof, as well as optionally also gold and/or gold compounds, and as activators alkali metal compounds and/or alkaline earth metal compounds and/or cadmium compounds on a carrier, characterized in that the carrier consists of rods having a starshaped cross section or of ribbed rods.

2. A catalyst as claimed in claim 1, characterized in that the cross section of the rods represent a 4- to 6-pointed star.

3. A catalyst as claimed in claims 1 or 2, characterized in that the carrier consists of silicic acid.

0 004 079

FIG.1

FIG.2

FIG.3

FIG.4

FIG.5

FIG.6

FIG.7

FIG. 8

1

FIG.1

FIG.2

FIG.3

FIG.4

FIG.5

FIG.6

FIG.7

FIG.8